# EUROPÄISCHE PATENTANMELDUNG

(11) **EP 0 615 277 A2**
(43) Veröffentlichungstag der Anmeldung: **14.09.1994**
(21) Anmeldenummer: 94101497.9
(22) Anmeldetag: 01.02.1994
(51) Int. Cl.: H01J 61/52

(54) **UV-Hochleistungsröhre**

(30) Priorität: 02.02.1993 DE 4302852
(71) Anmelder: IMAB-STIFTUNG, FL-9496 Balzers (LI)
(72) Erfinder: Brück, Gernot K., D-50858 Köln (DE)
(74) Vertreter: Lippert, Hans-Joachim, Dipl.-Ing.

(57) **Zusammenfassung**

Um die Emissionsrate von UV-Röhren, die beispielsweise zu kosmetischen Zwecken eingesetzt werden, erhöhen zu können, wird eine begrenzte, partielle Kühlung der Röhre vorgeschlagen. Dadurch ist trotz höherem Strominput der Gasdruck im Inneren der Röhre reduziert. Auch kann bei derartigen Röhren die Belegung mit UV-Leuchtpigmenten über ihre Länge unterschiedlich sein, so daß beispielsweise im Gesichtsbereich einer Bräunungsbank eine höhere UV-Strahlung emittiert wird.

## Beschreibung

### Stand der Technik

Sowohl im professionellen Bräunungsbereich, als auch zur privaten Verwendung finden sich viele Sonnenbänke, die überwiegend mit Quecksilberdampfniederdruckröhren ausgerüstet sind. Der innere Belag dieser Röhren sind spezielle Phosphore, die eine Energieumsetzung in der Weise vornehmen, daß die energiereichere Strahlung dieser Röhren in langwelligere umgesetzt wird.

Um eine höhere Bräunungsleistung zu erreichen werden in die Röhre stärkere Elektroden eingeschmolzen und dann durch einen stärkeren Stromfluß auch die Lichtleistung erhöht. Hierbei entstehen jedoch beachtliche thermische Probleme. Die Röhrenwände heizen sich stark auf, verbunden mit einem vermehrten langwelligen IR-Strahlung, wogegen der höhere Dampfdruck auch eine erhebliche Zunahme der kurzwelligeren IR-Strahlung verursacht.

Nur durch eine Zwangskühlung lassen sich solche Röhren betreiben. Üblicherweise wird ein starker Luftstrom entlang der Röhre geführt.

Da die Bräunungsleistung der UV-Röhren über die gesamte Länge mehr oder weniger gleich ist, aber im Gesichtsbereich eine wesentlich höhere Leistung erforderlich ist, werden in den meisten Bräunungsgeräten separate Gesichtsbräuner mit Hochdruckstrahlern eingebaut. Hierzu muß man wissen, daß das Gesicht, welches immer der täglichen UV-Bestrahlung der Sonne ausgesetzt ist, wesentlich uv-unempfindlicher ist als andere Körperteile.

Durch den Einbau der Gesichtbräuner wird die Besonnungsanlage wesentlich aufwendiger, komplizierter und teurer.

### Verfahren zur Leistungssteigerung der UV-Röhren zur Hochleistungsröhre

Bekanntlich richtet sich der Dampfdruck innerhalb einer Hg-Niederdruckröhre nach der tiefsten Temperatur an einer Stelle der Röhre.

Aus diesem Grunde ist es nicht erforderlich die gesamte Länge der Röhre zu kühlen, sondern es reicht aus dies punktuell vorzunehmen.

Erfindungsgemäß erfolgt dies durch eine Luftrohr mittels dem die Luftzufuhr auf einen Punkt der Röhre konzentriert wird. Steht gekühlte Luft zur Verfügung, so wird diese durch das Luftrohr geblasen und damit eine deutlichere Abkühlung erreicht.

Eine andere erfindungsgemäße Dampfdruckreduktion wird durch einen Kühlblock aus sehr wärmeleitfähigem Material erreicht, in dem ein Teil der Röhre gelagert ist. Das Innere des Kühlblocks ist hohl, so daß hierdurch ein beliebiges Kühlmittel geleitet werden kann.

Ein weiteres erfindungsgemäßes Verfahren ist das Anbringen einer Manschette, die z.B. mittels eines Peltierelementes gekühlt werden kann.

Mittels eines weiteren erfindungsgemäßen Verfahrens wird das Innere des in die Röhre hineinragenden Glasstutzens, in dem die Elektroden eingeschmolzen sind, durch Kühlluft auf geringere Temperatur gebracht. Auch hierdurch wird die Reduzierung des Dampfdrucks erreicht.

Ein weitere Abart des erfindungsgemäßen Verfahrens ist die Einschmelzung eines Stabes aus wärmeleitfähigem Material, wobei das aus der Röhre ragende Teil entweder über Kühlrippen oder mittels eines angesetzten Peltierelementes gekühlt wird und damit die Kühlung in das Innere der Röhre gebracht wird.

Da die Einschmelzung von fremden Materialien je nach Glassorte Schwierigkeiten verursachen kann, kann statt eines Stabs aus kompaktem Material auch eine u-förmige Glasröhre eingeschmolzen werden. Die Öffnungen dieser Glasröhre liegen vorzugsweise im Bereich des Sockels, damit an dieser Stelle die Luftdurchleitung geschehen kann.

Neben der Steigerung der Bräunungsleistung durch höheren Strominput, kann auch eine bessere Auslegung der Leuchtpigmente die Bräunungsleistung erhöhen.

Wie schon zuvor ausgeführt ist es wichtig, daß im Gesichtbereich eine höhere Bräunungsleistung zur Verfügung steht.

Bei dem erfindungsgemäßen Verfahren zur Schaffung einer Hochleistungsröhre wird in diesem Fall Einfluß auf die Belegung des Röhreninneren mit Leuchtpigmenten genommen. Zur besseren Versorgung des Gesichtsbereiches mit Bräunungslicht, wird das Leuchtpigmentgemisch zum Gesicht hin stärker mit UV-B-Phosphoren beladen.

Ein weiteres erfindungsgemäßes Verfahren ist die produktionstechnisch einfacherer Bildung von Segmenten von Leuchtpigmenten mit unterschiedlicher Konzentration von UV-B-Phosphoren.

### Beschreibung

Die erfindungsgemäßen Verfahren werden anhand der Figuren 1 bis 14 dargestellt, wobei die Figuren 1 bis 12 Verfahren zur Reduzierung des Dampfdrucks darstellen, wird in den Figuren 13 und 14 das Verfahren zur lageorientierten Einstellung der Bräunungsleistung dargestellt.

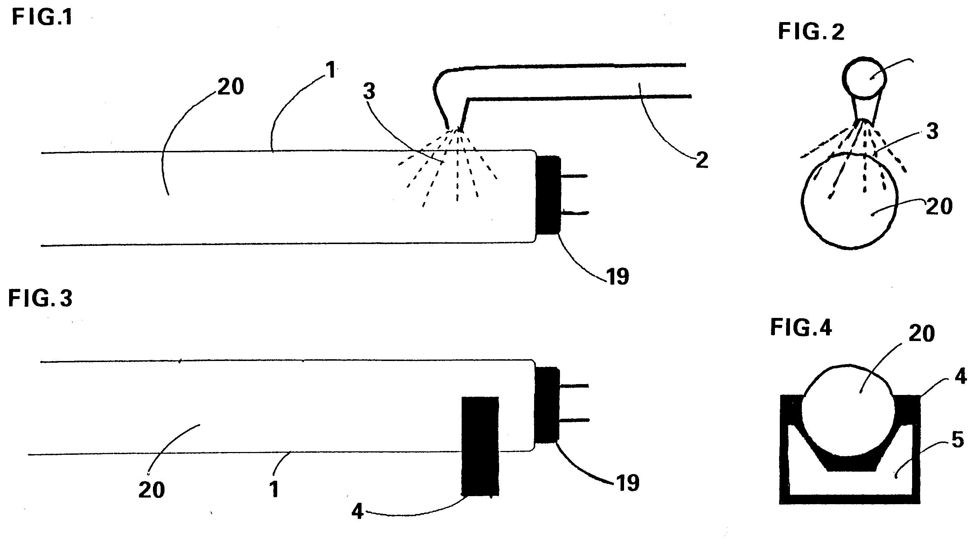

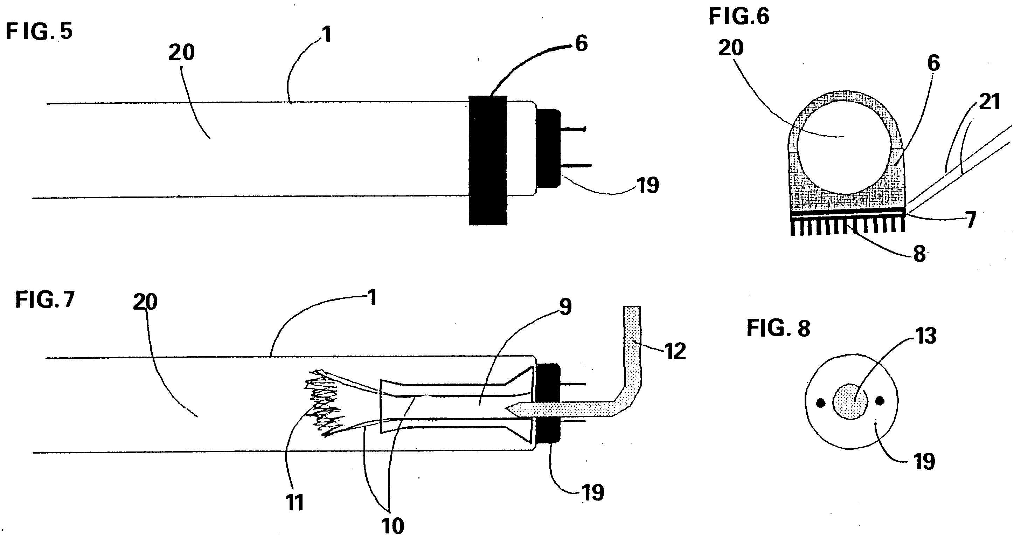

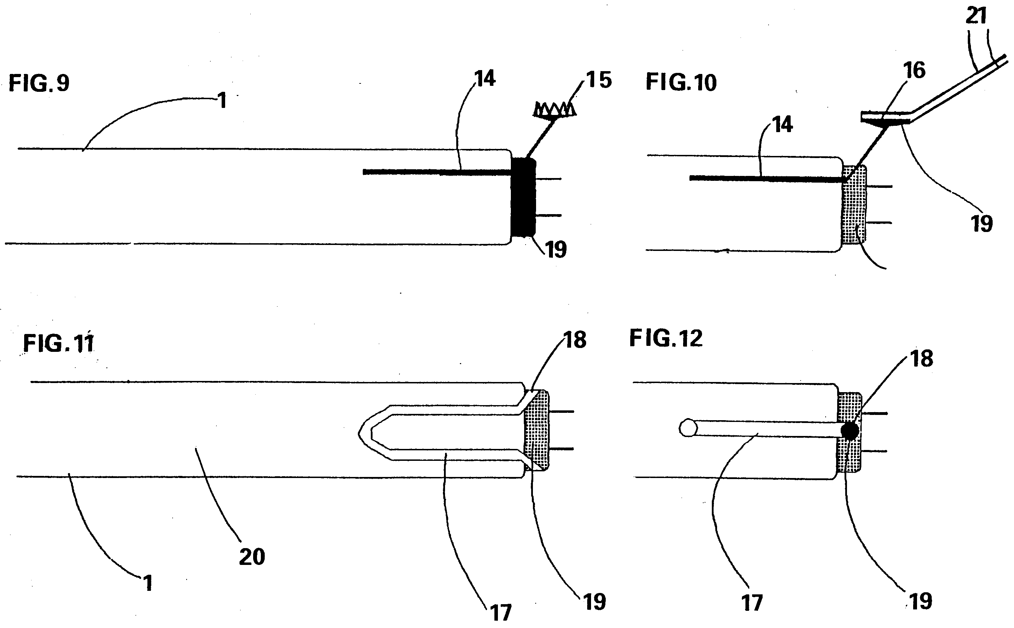

So sind in den Figuren 1 bis 12 die folgenden Einzelheiten dargestellt:
1.+2.) Das Röhrenglas 1 schließt die Röhre 20 nach außen ab. Über den durch die Luftröhre 2 zugeführten Luftstrom 3 wird die punktuelle Kühlung erreicht.
3.+4.) Die Röhre 20 liegt auf dem Sockel 4 aus wärmeleitfähigem Material auf, wobei dieser durch seine inneren Raum 5 mit Kühlmitteln versorgt werden kann.
5.+6.) Eine Manschette 6 aus ebenfalls wärmeleitfähigem Material umgibt das Rohr 20, wobei ein Peltierelement 7 mit Kühlrippen 8 zur Kühlung eingesetzt wird. Der notwendige Strom wird über die Drähte 21 zugeführt.
7.+8.) Der in die Röhre 20 hereinragende Stutzen 9 aus Glas, in dem die Elektrodenzuführungen 10 für die Elektrode 11 eingeschmolzen sind, wird über das Luftrohr 12 mit Kühlluft 3 gekühlt. Durch die Öffnung 13 im Sockel 19 kann das Luftrohr bis in den Stutzen 9 eingeführt werden.
9.+10.) Ein Stab 14 aus wärmeleitfähigem Material ist in die Röhre 20 mit eingeschmolzen, wobei an dem aus der Röhre herausragenden Teil entweder Kühlrippen 15 oder ein Peltierelement angebracht sein können.
11.+12.) Ein Glasrohr 17 mit den Öffnungen 18 im Sockel 19 ermöglicht die Kühlung des Röhren 20 - Innenraums.

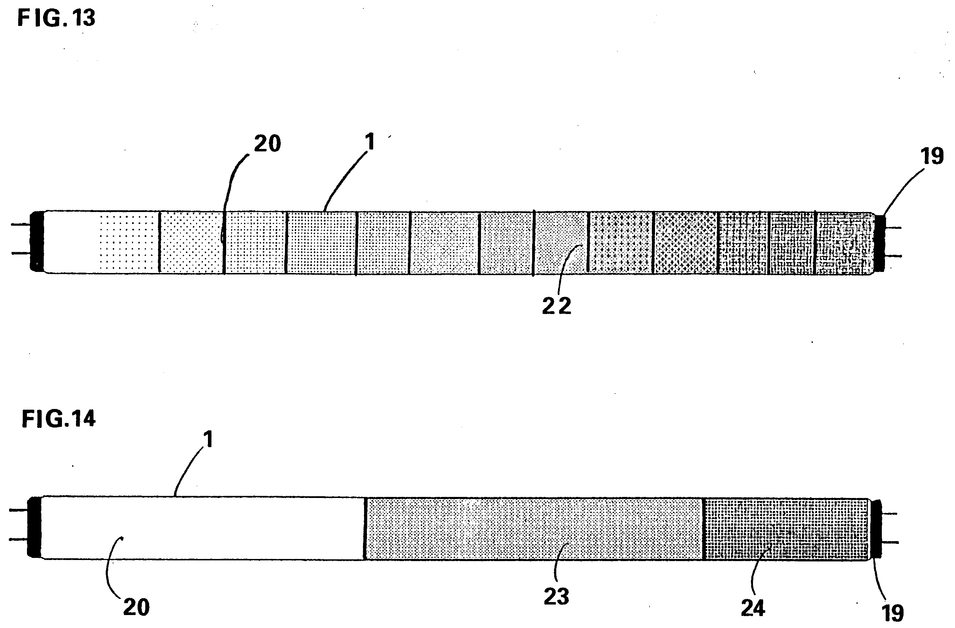

In den Figuren 13 und 14 sind die folgenden Einzelheiten dargestellt:
13.) In der Röhre 20 sind auf der Innenseite der Glaswandung 1 Leuchtpigmente 22 mit steigender Konzentration des UV-B-Leuchtpigmentes aufgetragen.
14.) Auf der Innenseite des Glases 1 können aber auch die Leuchtpigmentsegmente 23 und 24 mit jeweils unterschiedlicher Konzentration des UV-B-Leuchtpigmentes aufgetragen sein.

## Patentansprüche

1. Verfahren zur Schaffung einer UV-Hochleistungsröhre dadurch gekennzeichnet, daß der Gasdruck dadurch reduziert wird, indem die Röhre partiell mit Kühlung beaufschlagt wird.

2. Verfahren nach Anspruch 1 dadurch gekennzeichnet, daß die Röhre an einer Stelle mit stark gekühlter Luft angeblasen wird und dadurch an dieser Stelle die Fläche des Glaskörpers stark gekühlt wird.

3. Verfahren nach Anspruch 1 dadurch gekennzeichnet, daß die Röhre an einer Stelle auf einem sehr wärmeleitfähigen Material aufliegt und diese Auflage stark gekühlt wird.

4. Verfahren nach Anspruch 3 dadurch gekennzeichnet, daß diese Auflage in Form einer Manschette ausgebildet ist.

5. Verfahren nach Ansprüchen 3 und 4 dadurch gekennzeichnet, daß die Kühlung des wärmeleitfähigen Materials durch Peltierelemente erfolgt.

6. Verfahren nach Anspruch 1 dadurch gekennzeichnet, daß Luft in den Glasstutzen eingeblasen wird, der die Elektrodenstifte aufnimmt.

7. Verfahren nach Anspruch 6 dadurch gekennzeichnet, daß gekühlte Luft eingeblasen wird.

8. Verfahren nach Anspruch 1 dadurch gekennzeichnet, daß ein separater Stab aus wärmeleitfähigem Material mit eingeschmolzen wird und über diesen die Kühlung in den Gasraum der Röhre gebracht wird.

9. Verfahren nach Anspruch 8 dadurch gekennzeichnet, daß dieser Stab außerhalb der Röhre mit Kühlrippen versehen ist.

10. Verfahren nach Anspruch 8 dadurch gekennzeichnet, daß dieser Stab mit der Kühlseite eines Peltierelementes verbunden ist und die Kühlung elektrisch erfolgt..

11. Verfahren nach Anspruch 1 dadurch gekennzeichnet, daß im Bereich der Elektroden noch ein u-förmiges Glasrohr mit eingeschmolzen wird, dessen Öffnungen außerhalb der UV-Röhre liegen, durch welche Kühlluft geblasen werden kann.

12. Verfahren zur Schaffung einer UV-Hochleistungsröhre mit positionsabhängiger Bräunungsleistung dadurch gekennzeichnet, daß die Belegung mit UV-Leuchtpigmenten unterschiedlich ist.

13. Verfahren nach Anspruch 12 dadurch gekennzeichnet, daß zur Kopfseite hin ein Leuchtpigmentgemisch mit höherem UV-B-Phosphoranteil eingezogen wird.

14. Verfahren nach Anspruch 12 dadurch gekennzeichnet, daß die Leuchtpigmente in verschiedenen Segmenten aufgetragen wird.

15. Verfahren nach Anspruch 14. dadurch gekennzeichnet, daß die UV-B-Emission der Leuchtpigmentsegmente zum Kopf hin zunimmt.
